# EUROPÄISCHE PATENTSCHRIFT

(11) **EP 2 982 324 B1**
(45) Veröffentlichungstag und Bekanntmachung des Hinweises auf die Patenterteilung: **08.04.2020**
(21) Anmeldenummer: 14179974.2
(22) Anmeldetag: 06.08.2014
(51) Int. Cl.: A61B 18/12

(54) **Buchsenmodul, elektrochirurgisches Gerät und Set mit einem Buchsenmodul**
Socket module, electrosurgical device and set with a socket module
Module de douille, appareil électro-chirurgical et ensemble doté d'un module de douille

(43) Veröffentlichungstag der Anmeldung: 10.02.2016
(73) Patentinhaber: Erbe Elektromedizin GmbH, 72072 Tübingen (DE)
(72) Erfinder: Bopp, Benjamin, 72131 Ofterdingen (DE); Zimmermann, Torsten, 72144 Dusslingen (DE); Hauger, Armin, 72119 Ammerbuch (DE); Stocker, Jürgen, 72147 Nehren (DE)
(74) Vertreter: Bohnenberger, Johannes

(56) Entgegenhaltungen:
- EP-A1- 2 033 589
- EP-A2- 1 562 505
- EP-A2- 2 485 670
- WO-A1-2014/071184

## Beschreibung

Die Erfindung bezieht sich auf ein Buchsenmodul für ein elektrochirurgisches Gerät, ein elektrochirurgisches Gerät mit einem derartigen Buchsenmodul sowie ein Set mit einem Buchsenmodul und einem Entnahmewerkzeug.

Aus der Praxis sind elektrochirurgische Geräte, beispielsweise HF-Chirurgiegeräte, bekannt, die mehrere Buchsenmodule zum Anschluss von chirurgischen Instrumenten aufweisen. Die Buchsenmodule bilden Steckverbindungen zwischen den elektrochirurgischen Instrumenten und den Schaltkreisen im Inneren des elektrochirurgischen Geräts. Für den Anschluss von elektrochirurgischen Instrumenten und/oder Neutralelektroden sind aus der Praxis Buchsenmodule bekannt, die jeweils mehrere Anschlüsse für Instrumente mit verschiedenen Steckern aufweisen. Bei diesen bekannten Buchsenmodulen sind die Anschlüsse im Inneren des Gehäuses des jeweiligen Moduls miteinander verdrahtet, was zeitaufwendige Lötprozesse erfordert. Damit sind die Montagezeiten für derartige Buchsenmodule lang und zusätzlich muss sichergestellt werden, dass die Lötverbindungen den Anforderungen an die elektrischen Geräte, insbesondere bezüglich Temperaturschwankungen, während der gesamten Lebensdauer der Geräte standhalten.

WO 2004/045441 A2 beschreibt ein Buchsenmodul, bei dem die Anschlüsse mit einer Platine durch Blattfederkontakte verbunden sind.

Die Aufgabe der Erfindung besteht darin, ein Buchsenmodul für ein elektrochirurgisches Gerät anzugeben, bei dem die Montagezeiten verkürzbar sind und gleichzeitig die Qualität der elektrischen Verbindungen sichergestellt werden kann. Ferner besteht die Aufgabe der Erfindung darin, ein elektrochirurgisches Gerät mit einem solchen Buchsenmodul sowie ein Set mit einem Buchsenmodul und einem Entnahmewerkzeug anzugeben.

Mit Blick auf das Buchsenmodul wird die Aufgabe durch den Gegenstand des Anspruchs 1, mit Blick auf das elektrochirurgische Gerät durch den Gegenstand des Anspruchs 12 und mit Blick auf das Set durch den Gegenstand des Anspruchs 13 gelöst.

Die Erfindung beruht auf dem Gedanken, ein Buchsenmodul für ein elektrochirurgisches Gerät anzugeben, wobei das Buchsenmodul ein Gehäuse, wenigstens zwei Anschlüsse mit jeweils zwei Kontakten sowie wenigstens ein erstes Verbindungsmittel aufweist. Das erste Verbindungsmittel verbindet einen ersten Kontakt des ersten Anschlusses mit einem ersten Kontakt des zweiten Anschlusses elektrisch. Die Erfindung zeichnet sich dadurch aus, dass das erste Verbindungsmittel zwei Kontaktbereiche aufweist, von denen ein erster Kontaktbereich mit dem ersten Kontakt des ersten Anschlusses und ein zweiter Kontaktbereich mit dem ersten Kontakt des zweiten Anschlusses jeweils formschlüssig und/oder kraftschlüssig verbunden sind. Im Gehäuse ist eine Platine angeordnet, die mit dem ersten Anschluss elektrisch verbunden ist. Die Platine weist einen dritten Kontaktbereich auf, der den ersten Anschluss mit der Platine formschlüssig und/oder kraftschlüssig verbindet.

Die Erfindung hat verschiedene Vorteile. Durch die formschlüssige und/oder kraftschlüssige Verbindung entfällt zumindest teilweise die Verdrahtung der Anschlüsse, wodurch die Montagezeiten beträchtlich verkürzt werden. Außerdem werden potentielle Fehlerquellen, die beim Löten entstehen können, vermieden.

Die im Gehäuse angeordnete Platine trägt ebenso dazu bei, dass die Anzahl der Verbindungskabel im Gehäuse verringert wird, weil die Platine mit dem ersten Anschluss formschlüssig und/oder kraftschlüssig verbunden ist.

Im Zusammenhang mit dem ersten Verbindungsmittel wird konkret offenbart, dass der erste und zweite Kontaktbereich jeweils formschlüssig oder jeweils kraftschlüssig mit dem jeweiligen Kontakt verbunden sein kann. Es ist auch möglich, dass der erste Kontaktbereich formschlüssig und der zweite Kontaktbereich kraftschlüssig oder umgekehrt mit dem jeweiligen Kontakt verbunden ist.

Unter einer formschlüssigen Verbindung wird eine Verbindung zwischen zwei Verbindungsteilen verstanden, die zumindest teilweise ineinander greifen bzw. einander umgreifen. Dadurch können sich die Verbindungsteile auch ohne oder bei unterbrochener Kraftübertragung nicht lösen. Die formschlüssige Verbindung kann auch mit einer kraftschlüssigen Verbindung kombiniert sein, beispielsweise durch Beaufschlagen der jeweiligen Verbindungsteile mit einer Federkraft. Es sind rein kraftschlüssige Verbindungen möglich, bei denen die Haftreibung zwischen den Wirkflächen verhindert, dass sich die Teile voneinander lösen.

Die Kontaktbereiche sorgen sowohl für den mechanischen Halt der miteinander verbundenen Teile als auch für den elektrischen Kontakt.

Bei einer besonders bevorzugten Ausführungsform verbindet ein zweites Verbindungsmittel einen zweiten Kontakt des ersten Anschlusses mit einem zweiten Kontakt des zweiten Anschlusses elektrisch. Das zweite Verbindungsmittel weist zwei Kontaktbereiche auf, von denen ein erster Kontaktbereich mit dem zweiten Kontakt des ersten Anschlusses formschlüssig und/oder kraftschlüssig verbunden ist. Ein zweiter Kontaktbereich ist mit dem zweiten Kontakt des zweiten Anschlusses formschlüssig und/oder kraftschlüssig oder stoffschlüssig verbunden. Durch das zweite Verbindungsmittel wird der Verdrahtungsaufwand noch weiter verringert bzw. die Verdrahtung kann komplett entfallen. Im Vergleich zu den Montagezeiten bei bekannten Buchsenmodulen kann die Montagezeit bei dieser Ausführungsform um ca. 75 % verringert werden.

Zweckmäßigerweise ist wenigstens das erste Verbindungsmittel mit dem Gehäuse verbunden. Es ist auch möglich sowohl das erste als auch das zweite Verbindungsmittel mit dem Gehäuse zu verbinden, wodurch eine einfache Fixierung der Verbindungsmittel erreicht wird. Vorzugsweise ist das erste Verbindungsmittel mit einem abnehmbaren Deckel des Gehäuses verbunden, wodurch die Montagefreundlichkeit des Buchsenmoduls weiter verbessert wird, weil mit dem Schließen des Gehäuses durch den Deckel automatisch die Verbindung der jeweiligen Kontakte des ersten und zweiten Anschlusses erfolgt. Das zweite Verbindungsmittel kann mit einem Boden des Gehäuses verbunden sein und ist damit vom ersten Verbindungsmittel ausreichend sicher beabstandet.

Die Kontaktbereiche des ersten Verbindungsmittels können Clips und/oder Federzungen bilden, die mit den ersten Kontakten verrastet sind und/oder an diesen anliegen. Es bestehen also verschiedene Möglichkeiten, die Kontaktbereiche zu realisieren. Beispielsweise können der erste und zweite Kontaktbereich des ersten Verbindungsmittels jeweils durch Clips oder jeweils durch Federzungen ausgebildet sein. Es ist auch möglich, einen Kontaktbereich durch einen Clip und den anderen durch eine Federzunge auszubilden. Dies gilt für den ersten und zweiten Kontaktbereich bzw. umgekehrt. Die Verwendung von Clips hat den Vorteil, dass eine besonders sichere Verbindung hergestellt wird, die vom Anpressdruck des Deckels unabhängig ist. Der Vorteil der Federzungen besteht darin, dass diese besonders einfach herzustellen sind.

Die Kontaktbereiche des zweiten Verbindungsmittels können Clips und/oder Federzungen und/oder Anlageflächen bilden. Die Clips sind mit den jeweiligen zweiten Kontakten verrastet. Die Federzungen liegen an den zweiten Kontakten an und die Anlageflächen sind stoffschlüssig mit den zweiten Kontakten verbunden. Auch hier sind alle möglichen Kombinationen denkbar. Beispielsweise kann ein Kontaktbereich als Clip und der andere Kontaktbereich als Anlagefläche ausgebildet sein, die mit dem entsprechenden Kontakt stoffschlüssig, beispielsweise durch löten oder schweißen verbunden ist. Insbesondere wenn das zweite Verbindungsmittel am Boden des Gehäuses angeordnet ist, ist eine stoffschlüssige Verbindung zwischen dem jeweiligen Kontaktbereich und dem Kontakt einfach zu realisieren.

Bei einer bevorzugten Ausführung bildet wenigstens das erste Verbindungsmittel, insbesondere das erste und zweite Verbindungsmittel einen Blechstreifen, an dessen Enden die Kontaktbereiche vorgesehen sind. Der Blechstreifen ist einfach herzustellen und kann sicher und schnell im Gehäuse montiert werden.

Vorzugsweise bildet der dritte Kontaktbereich wenigstens einen Clip, der mit der Platine verbunden ist. Bei einer besonders bevorzugten Ausführungsform weist der dritte Kontaktbereich zwei Clips auf, die jeweils mit der Platine verbunden sind. Damit wird auf einfache Weise die bisher bekannte Verdrahtung der Anschlüsse durch eine mechanische, insbesondere eine Steck- bzw. Rastverbindung ersetzt.

Die Kontaktbereiche, insbesondere die Clips können zwei gegenüber angeordnete Backen aufweisen, die im Haltezustand mit einer Federkraft beaufschlagt sind. Dadurch wird eine sichere Fixierung der zwischen den Backen angeordneten Teile, insbesondere der Kontakte der Anschlüsse erreicht.

Zweckmäßigerweise weist der erste Anschluss einen Anschlag auf, der mit einer Außenkante der Platine zur Positionierung des Anschlusses zusammenwirkt. Die Platine hat damit eine Doppelfunktion. Zum einen dient sie dazu, die Anschlüsse mit den Schaltkreisen im Inneren des elektrochirurgischen Gerätes elektrisch zu verbinden. Zum anderen dient diese als mechanischer Anschlag, um den ersten Anschluss in Längsrichtung des Anschlusses korrekt zu positionieren. Dadurch wird die Montage des ersten Anschlusses erleichtert.

Der erste Anschluss kann zwei Stecker umfassen, die den ersten und zweiten Kontakt des ersten Anschlusses bilden. Eine derartige Ausbildung des Anschlusses ist beispielsweise für international verwendete Kabel geeignet.

Der zweite Anschluss kann eine Steckerbuchse und eine Klinkenfeder umfassen, wobei die Steckerbuchse den ersten Kontakt und die Klinkenfeder den zweiten Kontakt des zweiten Anschlusses bilden. Ein derartiger Anschluss ist für die Verbindung mit einem Klinkenstecker vorgesehen.

Der zweite Anschluss, insbesondere die Klinkenfeder kann auf wenigstens einen Haltestift aufgesteckt sein und durch wenigstens einen hülsenartigen Niederhalter fixiert sein, der den Haltestift nach der Montage umgibt. Zweckmäßigerweise sind zwei Haltestifte und zwei entsprechende Niederhalter vorgesehen. Die Fixierung des zweiten Anschlusses, insbesondere der Klinkenfeder durch den Haltestift und den Niederhalter ersetzt die an sich bekannte Schraubverbindung, mit der die Klinkenfeder im Gehäuse fixiert wird. Dadurch entfällt der gemäß Stand der Technik aufwendige Schraubprozess bei der Montage der Klinkenfeder. Vielmehr wird durch die Verbindung des Haltestifts mit dem hülsenartigen Niederhalter durch das Aufsetzen des Deckels die Verbindung automatisch hergestellt werden.

Die Erfindung wird nachfolgend mit weiteren Einzelheiten unter Bezug auf die beigefügten schematischen Zeichnungen näher erläutert. In diesen zeigen
- Fig. 1:: eine perspektivische Ansicht eines Buchsenmoduls nach einem erfindungsgemäßen Ausführungsbeispiel, bei dem der Deckel entfernt ist, wobei das erste Verbindungsmittel in der Einbaulage gezeigt ist;
- Fig. 2:: einen Querschnitt durch das Buchsenmodul gemäß Fig. 1 entlang der Linie A-A;
- Fig. 3:: einen Querschnitt durch das Gehäuse nach Fig. 1 entlang der Linie B-B; und
- Fig. 4:: einen Längsschnitt durch das Buchsenmodul gemäß Fig. 1 entlang der Linie C-C.

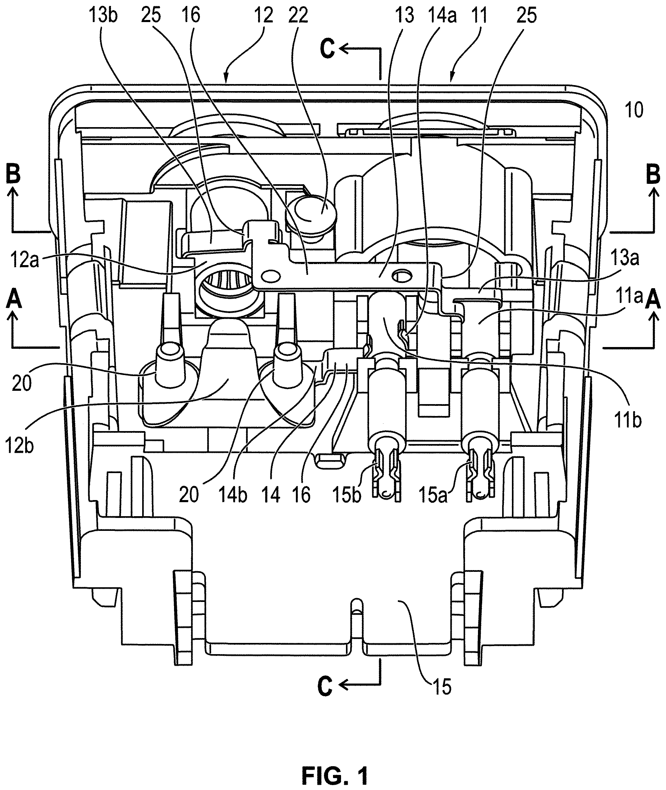

Das in Fig. 1 dargestellte Buchsenmodul kommt für den Anschluss von Neutralelektroden an ein elektrochirurgisches Gerät zum Einsatz (NE-Buchsenmodul). Die Erfindung ist nicht auf sogenannte NE-Buchsenmodule für Neutralelektroden eingeschränkt, sondern ist auch auf andere Buchsenmodule, mit denen elektrochirurgische Instrumente mit Geräten verbunden werden, anwendbar, wobei verschiedene Anschlüsse im Gehäuse des Buchsenmoduls miteinander elektrisch verbunden sind.

Das in Fig. 1 dargestellte Buchsenmodul ist besonders gut in Kombination mit dem in EP 14 154 490 beschriebenen Buchseneinsatz verwendbar, der werkzeuglos montiert werden kann und auf die Anmelderin zurückgeht.

Zu den weiteren Einzelheiten des werkzeuglos montierbaren Buchseneinsatzes bzw. Buchsenmoduls wird auf EP 14 154 490 verwiesen.

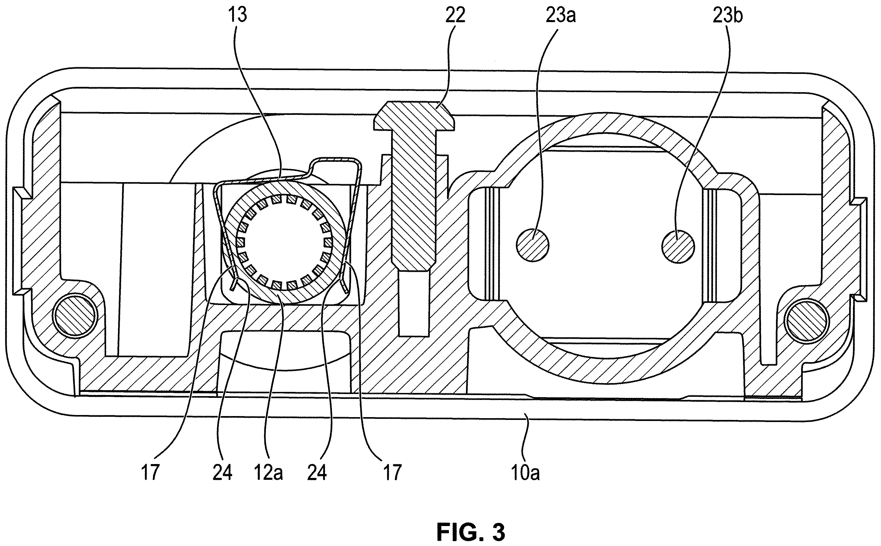

Das Buchsenmodul gemäß Fig. 1 weist ein Gehäuse 10 mit einem Boden 10a und einem Deckel auf, der abnehmbar ist und in Fig. 1 nicht dargestellt wird. Der Deckel kann durch die in den Figuren 1-4 dargestellte Schraube 22 fixiert werden. Andere Fixierungsmöglichkeiten wie beispielsweise Schnappverbindungen des Deckels sind denkbar. Das Gehäuse 10 weist zwei Anschlüsse 11, 12 auf. Die beiden Anschlüsse 11, 12 sind für unterschiedliche Steckertypen geeignet. Im Betrieb ist immer nur ein Anschluss belegt. Beispielsweise ist der erste Anschluss 11 für die Verbindung mit internationalen Kabeln ausgebildet und weist dazu elektrische Verbindungsteile, insbesondere zwei Kontaktstifte 23a ,23b auf, die in Fig. 3 dargestellt sind. Andere elektrische Verbindungsteile sind möglich.

Die Kontaktstifte 23a, 23b haben eine Doppelfunktion. Wenn der erste Anschluss 11 belegt ist, verbinden die Kontaktstifte 23a, 23b das angeschlossene Kabel mit einem elektronischen Bauteil, wie bspw. der Platine 15, im Gehäuseinneren. Wenn der zweite Anschluss 12 belegt ist, bilden die Kontaktstifte 23a, 23b Teil der elektrischen Verbindung zwischen dem zweiten Anschluss 12 und den weiteren Bauteilen, konkret der Platine 15. Gleichzeitig ist der erste Anschluss 11 blockiert, so dass nur am zweiten Anschluss 12 ein Kabel angeschlossen werden kann.

Für die elektrische Verbindung des zweiten Anschlusses 12 und der weiteren Bauteilen, konkret der Platine 15, bilden die elektrischen Verbindungsteile, insbesondere die Kontaktstifte 23a, 23b den ersten Kontakt 11a und den zweiten Kontakt 11b des ersten Anschlusses 11.

Die Kontaktstifte 23a, 23b sind als stiftförmige Drehteile ausgeführt.

Der in Fig. 1 dargestellte zweite Anschluss 12 ist für einen Klinkenstecker vorgesehen und weist dementsprechend eine Steckerbuchse 12a sowie eine Klinkenfeder 12b auf, die als erster Kontakt 12a bzw. als zweiter Kontakt 12b des zweiten Anschlusses 12 ausgebildet sind. Die Steckerbuchse wird nachfolgend mit dem Bezugszeichen 12a und die Klinkenfeder mit dem Bezugszeichen 12b bezeichnet.

Die beiden Anschlüsse 11, 12 sind, wie in Fig. 1 dargestellt, parallel nebeneinander Im Gehäuse 10 angeordnet. Die beiden Anschlüsse 11, 12 sind durch entsprechende Öffnungen in der Frontseite des Buchsenmoduls zugänglich. Bei dem Ausführungsbeispiel gemäß Fig. 1 ist eine Sicherung in Form einer Schiebeplatte vorgesehen, mit der einer der beiden Anschlüsse 11, 12 verschlossen wird, wenn der andere Anschluss 11, 12 zugänglich ist, so dass vermieden wird, dass beide Anschlüsse gleichzeitig belegt werden.

Wie in Fig. 1 gut zu erkennen, ist der erste Kontakt 11a des ersten Anschlusses 11, d.h. der Kontaktstift 23a mit dem ersten Kontakt 12a des zweiten Anschlusses 12, d.h. der Steckerbuchse 12a elektrisch verbunden. Dazu ist ein erstes Verbindungsmittel 13 in Form eines Blechstreifens 16 vorgesehen, der zwei Kontaktbereiche 13a, 13b aufweist. Der erste Kontaktbereich 13a ist mit dem Kontaktstift 23a und der zweite Kontaktbereich 13b mit der Steckerbuchse 12a mechanisch verbunden. Die Verbindung erfolgt als kombinierte form- und kraftschlüssige Verbindung, weil die Kontaktbereiche 13a, 13b den Kontaktstift 23a bzw. die Steckerbuchse 12a hintergreifen und zusätzlich mit einer Haltekraft, insbesondere einer Federkraft beaufschlagt sind, die für eine permanente Anlage der Kontaktbereiche 13a, 13b am Kontaktstift 23a, bzw. der Steckerbuchse 12a sorgt.

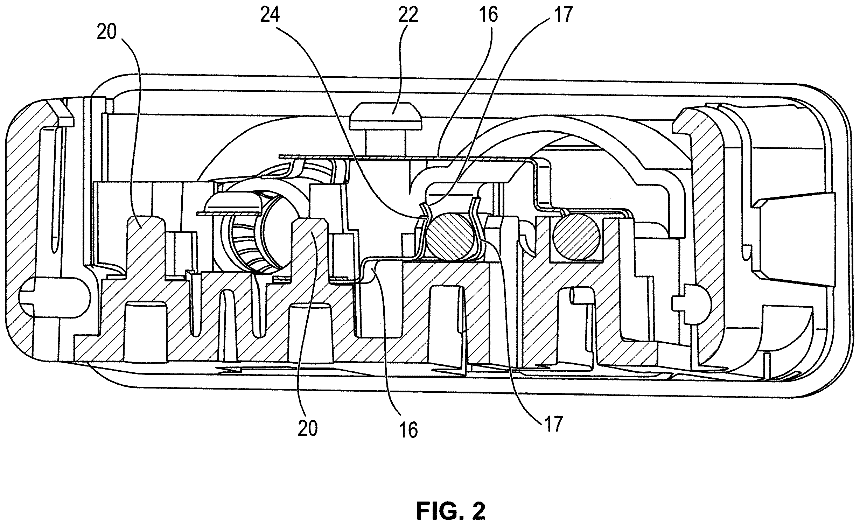

Konkret sind die Kontaktbereiche 13a, 13b jeweils als Clips 13a, 13b ausgeführt, die mit dem Kontaktstift 23a bzw. mit der Steckerbuchse 12a verrastet sind. Dazu weisen die Clips jeweils Backen 17 auf (Figur 2), die den ersten Kontaktstift 23a bzw. die Steckerbuchse 12a zumindest teilweise umgreifen. Die Backen 17 werden im montierten Zustand durch den Kontaktstift 23a bzw. die Steckerbuchse 12a auseinandergedrückt, so dass diese im montierten Zustand auf das jeweilige Bauteil, d.h. den Kontaktstift 23a bzw. die Steckerbuchse 12a eine Federkraft ausüben. Die Backen 17 sind so profiliert, dass diese den Kontaktstift 23a bzw. die Steckerbuchse 12a zumindest teilweise hintergreifen und somit zusätzlich zur kraftschlüssigen Verbindung auch einen gewissen Formschluss herstellen.

Der Abstand der beiden Backen 17 richtet sich nach dem Durchmesser des Kontaktstifts 23a bzw. der Steckerbuchse 12a. Dasselbe gilt für die Profilierung der Endstücke der beiden Backen 17, die bis zu einer Haltekante 24 nach Innen geneigt sind. Die Haltekante 24 hintergreift den Kontaktstift 23a bzw. die Steckerbuchse 12a, so dass zusammen mit der durch die Backen 17 aufgebrachten Federkraft eine sichere Verbindung hergestellt wird. Für die einfache Montage sind die Backen 17 jeweils nach der Haltekante 24 auseinander gebogen, so dass diese beim Aufstecken während der Montage nicht mit dem Kontaktstift 23a bzw. der Steckerbuchse 12a verkanten.

Die beiden Backen 17 für den ersten Kontaktstift 23a sind jeweils dadurch gebildet, dass der Blechstreifen 16 am entsprechenden Ende in Längsrichtung geteilt ist, wobei eine erste Backe 17 durch Umbiegen und Profilieren des geteilten Blechstreifens 16 gebildet wird. Dasselbe gilt für die zweite Backe 17, die in Längsrichtung beabstandet von der ersten Backe 17 gebildet ist. Wie in Fig. 1 gut zu sehen, sind die Backen 17 durch die Teilung des Blechstreifens 16 in Längsrichtung versetzt ausgebildet.

Das in Fig. 3 gezeigte Eintauchen der linken Backe 17 in das Material der Steckerbuchse 12a ist nur zeichnerisch bedingt. In der Praxis liegen beiden Backen an der Außenseite der Steckerbuchse 12b an. Dasselbe gilt für die anderen Backe 17, die den ersten Kontaktstift 23a umgreift.

Der Blechstreifen 16 weist im Bereich des ersten Kontaktstifts 23a eine Abkantung 25 auf, wodurch der Höhenunterschied zwischen dem ersten Kontaktstift 23a und dem Deckel ausgeglichen wird. Dasselbe gilt für den Blechstreifen 16 im Bereich der Steckerbuchse 12a, der dort eine entsprechende Abkantung 25 aufweist. Im montierten Zustand liegt der Abschnitt des Blechstreifens 16 zwischen den beiden Abkantungen 25 am Deckel an bzw. an der Innenseite des Deckels an. Zur Fixierung des Blechstreifens 16 ragen zwei Stifte des Deckels durch die in Fig. 1 dargestellten Öffnungen in den Blechstreifen 16.

Der Blechstreifen 16 ist im eingebauten Zustand parallel zum Boden bzw. parallel zum Deckel abgewinkelt, wobei ein langer Schenkel des Blechstreifens 16 senkrecht zu der Längsachse des ersten Kontaktstifts 23a und ein kürzerer Schenkel des Blechstreifens 16 parallel zur Längsachse der Steckerbuchse 12a angeordnet ist. Der erste Kontaktbereich 13a ist quer zur Längsachse des ersten Kontaktstifts 23a angeordnet. Dasselbe gilt für den zweiten Kontaktbereich 13b, der quer zur Längsachse der Steckerbuchse 12a angeordnet ist. Der zweite Kontaktbereich 13b ist mit dem kürzeren Schenkel des Blechstreifens 16 verbunden. Der erste Kontaktbereich 13a ist mit dem längeren Schenkel des Blechstreifens 16 verbunden.

Der Blechstreifen 16 bildet die elektrische Verbindung zwischen dem ersten und zweiten Kontakt bzw. der Steckerbuchse 12a und dem ersten Kontaktstift 23a.

Die Verbindung des ersten Kontaktstifts 23a mit der im Gehäuse angeordneten Platine 15 (siehe Fig. 1) erfolgt ebenfalls drahtlos. Dazu weist die Platine 15 einen dritten Kontaktbereich bestehend aus den Clips 15a, 15b auf, der den ersten Anschluss 11, konkret den ersten Kontaktstifts 23a form- und kraftschlüssig verbindet. Dazu ist auf der Platine 15 ein erster Clip 15a befestigt, insbesondere verlötet, der einerseits mit einem stiftartigen Ende des ersten Kontaktstifts 23a und andererseits mit einer in Fig. 1 nicht dargestellten Leiterbahn der Platine verbunden ist. Der Clip 15a umgreift das stiftartige Ende des ersten Kontaktstifts 23a. Der Clip 15a weist dazu zwei Backen 17 auf, die, wie oben beschrieben, profiliert sind. Insofern werden die im Zusammenhang mit dem Clip 13b für die Steckerbuchse 12a beschriebenen Merkmale in Bezug genommen.

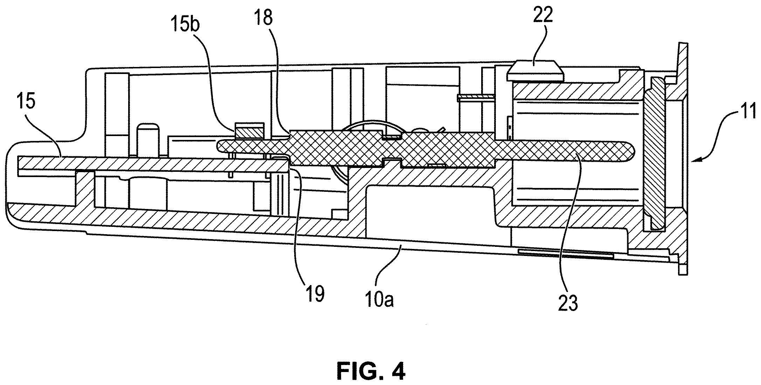

Das stiftartige Ende des ersten Kontaktstifts 23a weist einen kleineren Durchmesser als der Kontaktstift 23a auf. Dadurch wird eine einfache Verbindung mit relativ geringer Montagekraft zwischen dem ersten Anschluss bzw. dem ersten Kontaktstift 23a und der Platine 15 erreicht. Zur Vereinfachung der Positionierung des ersten Kontaktstifts 23a weist dieser einen Anschlag 18 auf, der im Einbauzustand mit einer Außenkante 19 der Platine 15 zusammenwirkt (Figur 4). Dadurch lässt sich der erste Kontaktstift 23a beim Einschieben oder Einclippen in Längsrichtung sicher positionieren. Dasselbe gilt für den zweiten Kontaktstift 23b , der den zweiten Kontakt 11b des ersten Anschlusses 11 bildet. Alle im Zusammenhang mit dem ersten Kontaktstift 23a und der Verbindung zur Platine 15 beschriebenen Merkmale werden auch im Zusammenhang mit dem zweiten Kontaktstift 23b offenbart.

Der zweite Kontaktstift 23b ist mit dem zweiten Kontakt 12b des ersten Anschlusses, konkret mit der Klinkenfeder 12b elektrisch verbunden. Die Klinkenfeder 12b ist in an sich bekannter Weise mit der Steckerbuchse 12a ausgerichtet und von dieser beabstandet, so dass ein zweipoliger Klinkenstecker mit dem zweiten Anschluss 12 verbunden werden kann. Die elektrische Verbindung zwischen der Klinkenfeder 12b und dem zweiten Kontaktstift 23b erfolgt durch ein zweites Verbindungsmittel 14. Das zweite Verbindungsmittel 14 ist als Blechstreifen 16 ausgeführt, der mit dem Boden 10a des Gehäuses 10 verbunden ist bzw. am Boden 10a angeordnet ist. Der Blechstreifen 16 bzw. das zweite Verbindungsmittel 14 weist einen ersten Kontaktbereich 14a auf, der mit dem zweiten Kontaktstift 23b form- und kraftschlüssig verbunden ist. Der erste Kontaktbereich 14a des zweiten Verbindungsmittels 14 ist bei dem Beispiel gemäß Fig. 1 als Clip analog zu dem Clip des ersten Verbindungsmittels 13 ausgeführt. Alle im Zusammenhang mit dem Clip des ersten Verbindungsmittels 13 offenbarten Merkmale werden auch im Zusammenhang mit dem Clip des zweiten Verbindungsmittels 14 offenbart und beansprucht. Die Clips sind entsprechend aufgebaut. Der einzige Unterschied zwischen den beiden Clips besteht darin, dass der Clip des zweiten Verbindungsmittels 14 ausgehend vom Boden 10a nach oben geöffnet ist, wohingegen der Clip des ersten Verbindungsmittels 13 nach unten geöffnet ist, also zum Boden 10a hin geöffnet ist. Die Öffnungsrichtung ergibt sich einfach daraus, dass das zweite Verbindungsmittel 14 am Boden 10a angeordnet ist, also dass der zweite Kontaktstift 23b von oben in den Clip eingeführt werden muss. Die Öffnungsrichtung des Clips des ersten Verbindungsmittels 13 ist durch die Anordnung des ersten Verbindungsmittels 13 am Deckel bedingt, so dass beim Aufsetzen des Deckels eine Verbindung des Clips des ersten Verbindungsmittels 13 mit dem ersten Kontaktstift 23a erfolgt.

Die Verbindung des zweiten Verbindungsmittels 14 mit der Klinkenfeder 12b erfolgt durch eine Anlagefläche, die den zweiten Kontaktbereich 14b bildet. Die Anlagefläche liegt an der Unterseite der Klinkenfeder an und ist mit dieser entweder kraftschlüssig durch eine von oben auf die Klinkenfeder wirkende Haltekraft oder stoffschlüssig durch Verschweißen oder Verlöten der Unterseite der Klinkenfeder mit dem zweiten Kontaktbereich 14b bewirkt.

Die Befestigung der Klinkenfeder 12b am Boden 10a des Gehäuses 10 erfolgt durch zwei Haltestifte 20, die auch Dome genannt werden. Im montierten Zustand wirken die beiden Haltestifte 20 mit nicht dargestellten hülsenartigen Niederhaltern zusammen, die am abnehmbaren Deckel befestigt sind. Die Niederhalter umgeben den jeweiligen Haltestift 20 und drücken die zwischen dem Niederhalter und dem Boden 10a angeordnete Klinkenfeder bzw. die beiden Laschen der Klinkenfeder nach unten gegen den Boden 10a. Auf eine Schraubverbindung kann daher zur Fixierung der Klinkenfeder 12b verzichtet werden. Auch dadurch wird der Montageaufwand verringert.

Insgesamt ermöglicht das Ausführungsbeispiel gemäß Fig. 1 einen vollständig drahtlosen Buchsenaufbau. Es ist auch möglich, nur einen Teil der Verdrahtung durch die mechanischen, d.h. nicht stoffschlüssigen Verbindungen zu ersetzen. Beispielsweise kann nur das obere erste Verbindungsmittel 13 verwendet werden, wohingegen die Verbindung zwischen dem zweiten Kontaktstift 23b und der Klinkenfeder 12b anderweitig gestaltet ist. Anstelle der in Fig. 1 dargestellten Clipverbindungen können andere mechanische Verbindungen, beispielsweise Federzungen vorgesehen sein, die die Clips einzeln oder insgesamt ersetzen. Die erforderliche Anpresskraft bei den Federzungen wird durch eine Verspannung des Deckels mit dem Gehäuse erreicht. Anstelle der Clipverbindung des zweiten Verbindungsmittels 14 kann ebenfalls eine Federzunge verwendet werden, die unten an dem zweiten Kontaktstift 23b anliegt.

Mit dem Ausführungsbeispiel gemäß Fig. 1 ist eine vollständig werkzeuglose Montage des Buchsenmoduls möglich.

Da das Buchsenmodul, wie in EP 14 154 490 einfach ausgetauscht werden kann, insbesondere ohne eine nachfolgende sicherheitstechnische Kontrolle, wird das Buchsenmodul gemäß Fig. 1 auch im Zusammenhang mit einem Set zusammen mit einem Entnahmewerkzeug offenbart und beansprucht, das wie beispielsweise in der vorstehend genannten europäischen Patentanmeldung näher beschrieben wird.

### Bezugszeichenliste

- 10: Gehäuse
- 10a: Boden des Gehäuses
- 11: erster Anschluss
- 11a: erster Kontakt (Verbindung)
- 11b: zweiter Kontakt (Verbindung)
- 12: zweiter Anschluss
- 12a: erster Kontakt (Steckerbuchse)
- 12b: zweiter Kontakt (Klinkenfeder)
- 13: erstes Verbindungsmittel
- 13a: erster Kontaktbereich (Clip)
- 13b: zweiter Kontaktbereich (Clip)
- 14: zweites Verbindungsmittel
- 14a: erster Kontaktbereich (Clip)
- 14b: zweiter Kontaktbereich (Anlagefläche)
- 15: Platine
- 15a, b: dritter Kontaktbereich, Kontaktmittel (Clips)
- 16: Blechstreifen
- 17: Backen
- 18: Anschlag
- 19: Außenkante
- 20: Haltestift
- 21: frei
- 22: Schraube
- 23a, 23b: Kontaktstifte
- 24: Haltekante
- 25: Abkantung

## Patentansprüche

1. Buchsenmodul für ein elektrochirurgisches Gerät, wobei das Buchsenmodul ein Gehäuse (10), wenigstens zwei Anschlüsse (11, 12) mit jeweils zwei Kontakten (11a, 11b, 12a, 12b) sowie wenigstens ein erstes Verbindungsmittel (13) aufweist, das einen ersten Kontakt (11a) des ersten Anschlusses (11) mit einem ersten Kontakt (12a) des zweiten Anschlusses (12) elektrisch verbindet,
**dadurch gekennzeichnet, dass**
- das erste Verbindungsmittel (13) zwei Kontaktbereiche (13a, 13b) aufweist, von denen ein erster Kontaktbereich (13a) mit dem ersten Kontakt (11a) des ersten Anschlusses (11) und ein zweiter Kontaktbereich (13b) mit dem ersten Kontakt (12a) des zweiten Anschlusses (12) jeweils formschlüssig und/oder kraftschlüssig verbunden sind, und
- eine Platine (15) im Gehäuse (10) angeordnet ist, die mit dem ersten Anschluss (11) elektrisch verbunden ist, wobei die Platine (15) einen dritten Kontaktbereich (15a, 15b) aufweist, der den ersten Anschluss (11) mit der Platine (15) formschlüssig oder kraftschlüssig verbindet, und
- ein zweites Verbindungsmittel (14) einen zweiten Kontakt (11b) des ersten Anschlusses (11) mit einem zweiten Kontakt (12b) des zweiten Anschlusses (12) elektrisch verbindet, wobei das zweite Verbindungsmittel (14) zwei Kontaktbereiche (14a, 14b) aufweist, von denen ein erster Kontaktbereich (14a) mit dem zweiten Kontakt (11b) des ersten Anschlusses (11) formschlüssig und/oder kraftschlüssig und ein zweiter Kontaktbereich (14b) mit dem zweiten Kontakt (12b) des zweiten Anschlusses (12) formschlüssig und/oder kraftschlüssig oder stoffschlüssig verbunden sind, und wobei das erste Verbindungsmittel (13) mit einem abnehmbaren Deckel des Gehäuses (10) und das zweite Verbindungsmittel (14) mit einem Boden (10a) des Gehäuses (10) verbunden sind.

2. Buchsenmodul nach Anspruch 1,
**dadurch gekennzeichnet, dass**
wenigstens das erste Verbindungsmittel (13), insbesondere das erste und zweite Verbindungsmittel (13, 14), mit dem Gehäuse (10) verbunden ist.

3. Buchsenmodul nach Anspruch 1 oder 2,
**dadurch gekennzeichnet, dass**
die Kontaktbereiche (13a, 13b) des ersten Verbindungsmittels (13) Clips und/oder Federzungen bilden, die mit den ersten Kontakten (11a, 12a) verrastet sind und/oder an diesen anliegen.

4. Buchsenmodul nach einem der vorhergehenden Ansprüche,
**dadurch gekennzeichnet, dass**
die Kontaktbereiche (14a, 14b) des zweiten Verbindungsmittels (14) Clips und/oder Federzungen und/oder Anlageflächen bilden, die mit den zweiten Kontakten (11b, 12b) verrastet sind und/oder an diesen anliegen und/oder stoffschlüssig mit diesen verbunden sind.

5. Buchsenmodul nach einem der vorhergehenden Ansprüche,
**dadurch gekennzeichnet, dass**
wenigstens das erste Verbindungsmittel (13), insbesondere das erste und zweite Verbindungsmittel (13, 14), einen Blechstreifen (16) bilden, an dessen Enden die Kontaktbereiche (13a, 13b, 14a, 14b) vorgesehen sind.

6. Buchsenmodul nach einem der vorhergehenden Ansprüche,
**dadurch gekennzeichnet, dass**
der dritte Kontaktbereich (15a, 15b) wenigstens einen Clip bildet, der mit der Platine (15) verbunden ist.

7. Buchsenmodul nach einem der vorhergehenden Ansprüche,
**dadurch gekennzeichnet, dass**
die Kontaktbereiche (13a, 13b, 14a, 14b, 15a, 15b), insbesondere Clips, zwei gegenüberangeordnete Backen (17) aufweisen, die im Haltezustand mit einer Federkraft beaufschlagt sind.

8. Buchsenmodul nach einem der vorhergehenden Ansprüche,
**dadurch gekennzeichnet, dass**
der erste Anschluss (11) einen Kontaktstift (23a, 23b) umfasst der einen Anschlag (18) aufweist, der mit einer Außenkante (19) der Platine (15) zur Positionierung des Anschlusses (11) zusammenwirkt.

9. Buchsenmodul nach einem der vorhergehenden Ansprüche,
**dadurch gekennzeichnet, dass**
der erste Anschluss (11) zwei Kontaktstifte (23a, 23b) umfasst, die den ersten und zweiten Kontakt (11a, 11b) des ersten Anschlusses (11) bilden.

10. Buchsenmodul nach einem der vorhergehenden Ansprüche,
**dadurch gekennzeichnet, dass**
der zweite Anschluss (12) eine Steckerbuchse und eine Klinkenfeder umfasst, wobei die Steckerbuchse den ersten Kontakt (12a) und die Klinkenfeder den zweiten Kontakt (12b) des zweiten Anschlusses (12) bilden.

11. Buchsenmodul nach einem der vorhergehenden Ansprüche,
**dadurch gekennzeichnet, dass**
der zweite Anschluss (12), insbesondere die Klinkenfeder, auf wenigstens einen Haltestift (20) aufgesteckt ist und durch wenigstens einen hülsenartigen Niederhalter fixiert ist, der den Haltestift (20) umgibt.

12. Elektrochirurgisches Gerät mit wenigstens einem Buchsenmodul nach Anspruch 1.

13. Set mit einem Buchsenmodul nach Anspruch 1 und einem Entnahmewerkzeug, das
wenigstens eine Entriegelungsstange mit einem freien Ende aufweist.

## Claims

1. Socket module for an electrosurgical device, wherein the socket module has a housing (10), at least two connectors (11, 12) each with two contacts (11a, 11b, 12a, 12b), and at least one first connection means (13) which electrically connects a first contact (11a) of the first connector (11) to a first contact (12a) of the second connector (12), **characterized in that**
- the first connection means (13) has two contact regions (13a, 13b), a first contact region (13a) of which is connected to the first contact (11a) of the first connector (11) and a second contact region (13b) of which is connected to the first contact (12a) of the second connector (12), in each case in a form-fitting and/or force-fitting manner, and
- a circuit board (15) is arranged in the housing (10) and is electrically connected to the first connector (11), wherein the circuit board (15) has a third contact region (15a, 15b) which connects the first connector (11) to the circuit board (15) in a form-fitting or force-fitting manner, and
- a second connection means (14) electrically connects a second contact (11b) of the first connector (11) to a second contact (12b) of the second connector (12), wherein the second connection means (14) has two contact regions (14a, 14b), a first contact region (14a) of which is connected to the second contact (11b) of the first connector (11) in a form-fitting and/or force-fitting manner and a second contact region (14b) of which is connected to the second contact (12b) of the second connector (12) in a form-fitting and/or force-fitting manner or with a material bond, and wherein the first connection means (13) is connected to a removable cover of the housing (10) and the second connection means (14) is connected to a base (10a) of the housing (10).

2. Socket module according to Claim 1,
**characterized in that**
at least the first connection means (13), in particular the first and second connection means (13, 14), is/are connected to the housing (10).

3. Socket module according to Claim 1 or 2,
**characterized in that**
the contact regions (13a, 13b) of the first connection means (13) form clips and/or spring tongues which are latched to the first contacts (11a, 12a) and/or rest against the latter.

4. Socket module according to one of the preceding claims,
**characterized in that**
the contact regions (14a, 14b) of the second connection means (14) form clips and/or spring tongues and/or contact surfaces which are latched to the second contacts (11b, 12b) and/or rest against the latter and/or are connected thereto with a material bond.

5. Socket module according to one of the preceding claims,
**characterized in that**
at least the first connection means (13), in particular the first and second connection means (13, 14), form(s) a metal strip (16), at the ends of which the contact regions (13a, 13b, 14a, 14b) are provided.

6. Socket module according to one of the preceding claims,
**characterized in that**
the third contact region (15a, 15b) forms at least one clip which is connected to the circuit board (15) .

7. Socket module according to one of the preceding claims,
**characterized in that**
the contact regions (13a, 13b, 14a, 14b, 15a, 15b), in particular clips, have two jaws (17) which are arranged opposite one another and to which a spring force is applied in the holding state.

8. Socket module according to one of the preceding claims,
**characterized in that**
the first connector (11) comprises a contact pin (23a, 23b) having a stop (18) which interacts with an outer edge (19) of the circuit board (15) in order to position the connector (11).

9. Socket module according to one of the preceding claims,
**characterized in that**
the first connector (11) comprises two contact pins (23a, 23b) which form the first and second contacts (11a, 11b) of the first connector (11).

10. Socket module according to one of the preceding claims,
**characterized in that**
the second connector (12) comprises a plug socket and a catch spring, wherein the plug socket forms the first contact (12a) and the catch spring forms the second contact (12b) of the second connector (12) .

11. Socket module according to one of the preceding claims,
**characterized in that**
the second connector (12), in particular the catch spring, is plugged onto at least one holding pin (20) and is fixed by means of at least one sleeve-shaped hold-down device which surrounds the holding pin (20).

12. Electrosurgical device having at least one socket module according to Claim 1.

13. Set having a socket module according to Claim 1 and a removal tool which has at least one unlocking rod with a free end.

## Revendications

1. Module de douille pour un instrument électrochirurgical, le module de douille possédant un boîtier (10), au moins deux bornes (11, 12) dotées respectivement de deux contacts (11a, 11b, 12a, 12b) ainsi qu'au moins un premier moyen de liaison (13) qui relie électriquement un premier contact (11a) de la première borne (11) à un premier contact (12a) de la deuxième borne (12),
**caractérisé en ce que**
- le premier moyen de liaison (13) possède deux zones de contact (13a, 13b), parmi lesquelles une première zone de contact (13a) est reliée au premier contact (11a) de la première borne (11) et une deuxième zone de contact (13b) est reliée au premier contact (12a) de la deuxième borne (12), respectivement par complémentarité de formes et/ou par assemblage de force, et
- une platine (15) est disposée dans le boîtier (10), laquelle est reliée électriquement à la première borne (11), la platine (15) possédant une troisième zone de contact (15a, 15b) qui relie la première borne (11) à la platine (15) par complémentarité de formes et par assemblage de force, et
- un deuxième moyen de liaison (14) relie électriquement un deuxième contact (11b) de la première borne (11) à un deuxième contact (12b) de la deuxième borne (12), le deuxième moyen de liaison (14) possédant deux zones de contact (14a, 14b), parmi lesquelles une première zone de contact (14a) est reliée au deuxième contact (11b) de la première borne (11) par complémentarité de formes et/ou par assemblage de force et une deuxième zone de contact (14b) est reliée au deuxième contact (12b) de la deuxième borne (12) par complémentarité de formes et/ou par assemblage de force ou par adhésion de matière, et le premier moyen de liaison (13) étant relié à un couvercle amovible du boîtier (10) et le deuxième moyen de liaison (14) à un fond (10a) du boîtier (10).

2. Module de douille selon la revendication 1, **caractérisé en ce que** le premier moyen de liaison (13), notamment le premier et le deuxième moyen de liaison (13, 14), est relié au boîtier (10).

3. Module de douille selon la revendication 1 ou 2, **caractérisé en ce que** les zones de contact (13a, 13b) du premier moyen de liaison (13) forment des agrafes et/ou des languettes flexibles qui sont enclipsées avec les premiers contacts (11a, 12a) et/ou qui reposent contre ceux-ci.

4. Module de douille selon l'une des revendications précédentes, **caractérisé en ce que** les zones de contact (14a, 14b) du deuxième moyen de liaison (14) forment des agrafes et/ou des languettes flexibles et/ou des surfaces d'appui qui sont enclipsées avec les deuxièmes contacts (11b, 12b) et/ou qui reposent contre ceux-ci et/ou qui sont reliés à ceux-ci par adhésion de matière.

5. Module de douille selon l'une des revendications précédentes, **caractérisé en ce que** le premier moyen de liaison (13), notamment le premier et le deuxième moyen de liaison (13, 14), forme une bande de tôle (16) aux extrémités de laquelle se trouvent les zones de contact (13a, 13b, 14a, 14b).

6. Module de douille selon l'une des revendications précédentes, **caractérisé en ce que** la troisième zone de contact (15a, 15b) forme au moins une agrafe qui est reliée à la platine (15).

7. Module de douille selon l'une des revendications précédentes, **caractérisé en ce que** les zones de contact (13a, 13b, 14a, 14b, 15a, 15b), notamment les agrafes, possèdent deux faces (17) disposées l'une en face de l'autre qui, à l'état de maintien, sont sollicitées par une force de ressort.

8. Module de douille selon l'une des revendications précédentes, **caractérisé en ce que** la première borne (11) comporte une broche de contact (23a, 23b), laquelle possède une butée (18) qui coopère avec une arête extérieure (19) de la platine (15) pour positionner la borne (11).

9. Module de douille selon l'une des revendications précédentes, **caractérisé en ce que** la première borne (11) comporte deux broches de contact (23a, 23b) qui forment les premier et deuxième contacts (11a, 11b) de la première borne (11).

10. Module de douille selon l'une des revendications précédentes, **caractérisé en ce que** la deuxième borne (12) comporte une douille à enfichage et un ressort de cliquet, la douille à enfichage formant le premier contact (12a) et le ressort de cliquet formant le deuxième contact (12b) de la deuxième borne (12).

11. Module de douille selon l'une des revendications précédentes, **caractérisé en ce que** la deuxième borne (12), notamment le ressort de cliquet, est enfichée sur au moins une broche de maintien (20) et bloquée par au moins un élément de retenue de type manchon qui entoure la broche de maintien (20).

12. Instrument électrochirurgical comprenant au moins un module de douille selon la revendication 1.

13. Ensemble composé d'un module de douille selon la revendication 1 et d'un outil de retrait qui possède au moins une tige de déverrouillage ayant une extrémité libre.
